# EUROPEAN PATENT SPECIFICATION

(11) **EP 3 342 363 B1**
(45) Date of publication and mention of the grant of the patent: **09.09.2020**
(21) Application number: 17199917.0
(22) Date of filing: 03.11.2017
(51) Int. Cl.: A61B 18/14, A61B 18/00

(54) **APPARATUS FOR ABLATION OF BODY TISSUE**
VORRICHTUNG ZUR ABLATION VON KÖRPERGEWEBE
APPAREIL D'ABLATION DE TISSU CORPOREL

(30) Priority: 02.12.2016 US 201615368296
(43) Date of publication of application: 04.07.2018
(73) Proprietor: Medtronic Holding Company Sàrl, 1131 Tolochenaz (CH)
(72) Inventor: AYVAZYAN, David R., Vallejo, CA 94590 (US); DUEIRI, David F., Santa Clara, CA 95051 (US)
(74) Representative: Viering, Jentschura & Partner mbB Patent- und Rechtsanwälte

(56) References cited:
- EP-A1- 1 299 044
- US-A1- 2007 250 054
- US-A1- 2015 126 922

## Description

### BACKGROUND OF THE INVENTION

### FIELD OF THE INVENTION

The present invention relates to a tumor ablation system and method for use thereof.

### DESCRIPTION OF THE PRIOR ART

Metastases are the most common cause of severe pain among patients with cancer. It has been reported that metastatic bone disease occurs in 60-80% of cancer patients.

Radiofrequency ablation is a method of treating metastatic bone disease. Radiofrequency ablation is used for the destruction of unwanted tissue, including tumors. During radiofrequency ablation, a probe is inserted into the unwanted tissue. A pair of electrodes are located at the end of the probe which is inserted into the unwanted tissue. The opposite end of the probe is connected to a radiofrequency generator which sends radiofrequency energy through the electrodes causing the immediately adjacent tissue to heat up. Once the unwanted tissue reaches a sufficient temperature for a specific period of time, the tissue dies. Radiofrequency ablation of a tumor takes about 20-30 minutes.

As an example, EP 1299044 A1 discloses a medical probe, comprising: an elongate member having a proximal end and a distal end, a plurality of temperature sensors carried by the distal end of the elongate member, and a common electrical bus carried by the elongate member, and defining two or more electrical paths, each of which is coupled to the plurality of temperature sensors.

As a further example, US 2015 126922 A1 discloses an energy delivery probe for use in tissue ablation, wherein the energy delivery device has at least a first energy delivery member and a second energy delivery member that have handle members positioned along a longitudinal axis, each handle member having a proximal and distal end. The distal end of the first handle member is releasably coupled to the proximal end of the second handle member and a portion of each member is defined in a coaxially surrounding relationship to each other along the longitudinal axis.

Currently available systems for radiofrequency ablation include probes having two electrodes located at the distal end of the probe. The currently available systems either have only one temperature sensor located near the distal end of the probe or rely on a separate temperature sensor that requires a separate incision for insertion into the patient. The currently available systems can be used with two probes. However, they do not transmit radiofrequency energy from one probe to the other. They merely insert two probes, each of which transmits radiofrequency energy between the two electrodes on that probe, and rely on the conduction of heat by the bone to ablate the bone between the two probe tips. Accordingly, the prior art systems are not capable of ablating irregular shapes or larger spaces without repositioning the probes.

### SUMMARY OF THE INVENTION

The present invention is directed to a system and method of radiofrequency tumor ablation that enables ablating irregular shapes by utilizing directional application of radiofrequency energy. The system includes a pair of probes wherein each of the probes includes two electrodes and three temperature sensors. The electrodes are connected to a radiofrequency generator configured to transmit radiofrequency energy between any pair of the four electrodes. The system may include channels through the probes, enabling the circulation of cooling fluid to help prevent singeing the tissue immediately adjacent the probes. The system may also include a grounding pad that includes a return electrode for placement outside the patient. The grounding pad may be used as a return electrode for any of the four electrodes.

The present invention provides a system for use in ablation according to claim 1. Further embodiments of the present invention are disclosed in the dependent claims.

The system further contemplates the radiofrequency generator including a processor programed to automatically adjust the amount of energy transmitted between the electrodes based on the temperature readings registered by the temperature sensors. The processor may also be programed to automatically change the pair of electrodes being utilized to achieve a specific ablation pattern.

A method for use in ablation includes making two incisions in the skin of the patient, inserting two cannulas through the incisions to abut the target bone, inserting a cutting tool through the cannulas to create openings in the bone, inserting two probes through the cannulas, attaching the probes to the radiofrequency generator, attaching the probes to the reservoirs, and applying radiofrequency energy between any pair of the electrodes.

A method for use in ablation, in accordance with one embodiment, includes making a first incision and a second incision in skin of a patient, creating a first opening and a second opening in a bone, inserting a first probe including a first electrode and a second electrode through the first incision and into the first opening, inserting a second probe including a third electrode and a fourth electrode through the second incision and into the second opening, attaching the first and second probes to a radiofrequency generator via a pair of cables, ablating tissue by applying radiofrequency energy between a first pair of electrodes, ablating additional tissue by applying radiofrequency energy between a second pair of electrodes, and removing the first and second probes.

The method further contemplates a processor automatically adjusting the amount of energy transmitted between the electrodes based on the temperature readings registered by the temperature sensors and automatically changing the pair of electrodes being utilized to achieve a specific ablation pattern.

These and other objects of the present invention will be apparent from review of the following specification and the accompanying drawings.

### BRIEF DESCRIPTION OF THE DRAWINGS

The accompanying drawings, which are incorporated in and constitute a part of the specification, illustrate preferred embodiments of the invention and, together with the description, serve to explain the objects, advantages, and principles of the invention.
FIG. 1 is a top plan view of a pair of probes for use in tumor ablation in a vertebral body;
FIG. 2 is a top plan view of the pair of probes for use in tumor ablation in accordance with an embodiment of the present invention showing radiofrequency energy being transmitted between two pairs of electrodes to achieve ablation of an irregularly shaped tumor;
FIG. 3 is an illustration of the distal ends of the pair of probes and a grounding pad including an electrode showing ten different possible pairings of electrodes between which radiofrequency energy may be applied; and
FIG. 4 is an illustration of the pair of probes connected to a radiofrequency generator and a peristaltic pump, a temperature sensor connected to the radiofrequency generator, and the grounding pad connected to the radiofrequency generator.

### DETAILED DESCRIPTION OF THE PREFERRED EMBODIMENT

The detailed description of the invention below is described for, and shown in the figures for, use in a vertebral body V. However, it should be understood that the invention could be used for tumor ablation in any bone.

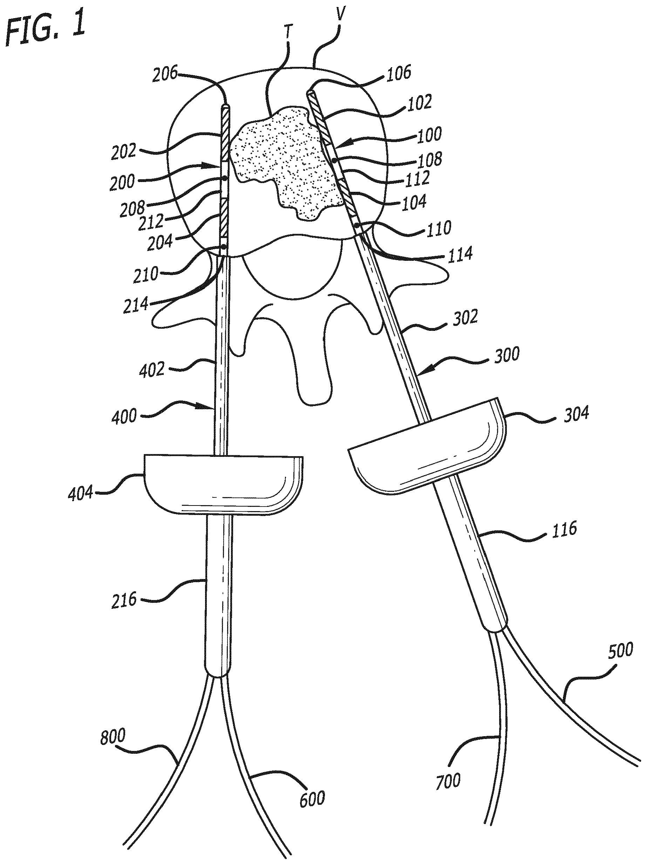

As shown in FIG. 1, the system for use in tumor ablation includes a pair of probes 100, 200. Each of probes 100, 200 includes a distal temperature sensor 106, 206 located near the distal end of probe 100, 200. Adjacent to distal temperature sensor 106, 206 is a distal electrode 102, 202. Each probe 100, 200 includes a proximal electrode 104, 204 separated from distal electrode 102, 202 by an insulated section 112, 212. Located on insulated section 112, 212 between distal electrode 102, 202 and proximal electrode 104, 204 is a midpoint temperature sensor 108, 208. The length of probe 100, 200 between the proximal electrode 104, 204 and a handle 116, 216 is an insulated shaft 114, 214. Located on insulated shaft 114, 214 adjacent to proximal electrode 104, 204 is a proximal temperature sensor 110, 210.

The temperature sensors 106, 108, 110, 206, 208, 210 may comprise thermocouples, resistive temperature detectors, thermistors, or any other suitable type of temperature sensing device. Electrodes 102, 104, 202, 204, 902 may be constructed of titanium, nitinol, steel, platinum, or any other biocompatible electrically conductive material.

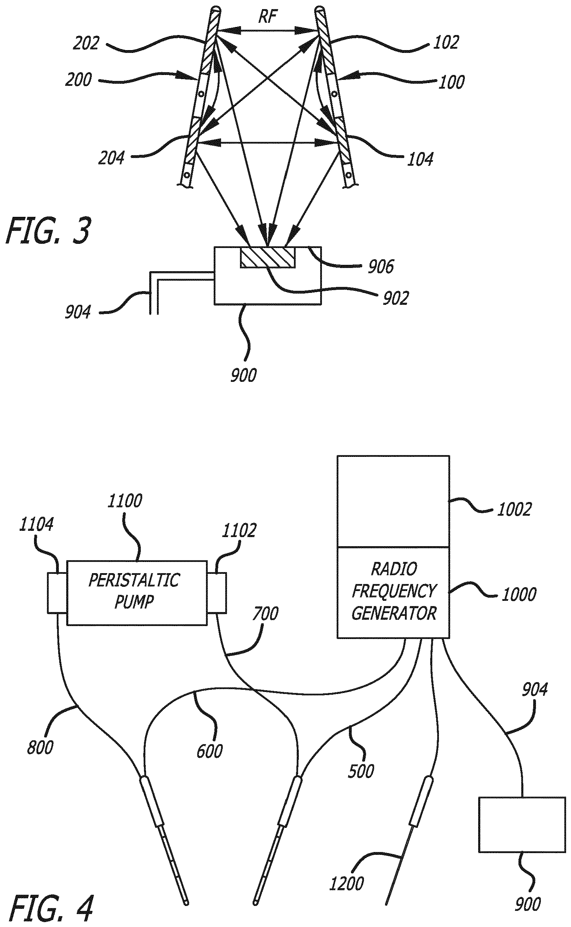

As shown in FIGS. 1 and 4, the probes 100, 200 are connected via cables 500, 600 to a radiofrequency generator 1000. The cables 500, 600 are configured to connect to the proximal end of handles 116, 216 and to radiofrequency generator 1000. Cables 500, 600 are configured to carry radiofrequency energy from the radiofrequency generator 1000 to electrodes 102, 104, 202, 204, and to carry temperature data from temperature sensors 106, 108, 110, 206, 208, 210 to a processor controlling radiofrequency generator 1000.

Probes 100, 200 also include an internal channel (not shown) that begins at the proximal end of handle 116, 216, runs the length of probe 100, 200, and returns to the proximal end of handle 116, 216 for pumping a cooling fluid therethrough, wherein the cooling fluid is preferably sterile water. The cooling fluid serves to cool the surface of the electrodes 102, 104, 206, 208 to prevent singeing of the tissue adjacent thereto. Probes 100, 200 may further include one or more radiopaque markers adjacent electrodes 102,104, 202, 204 to aid in the visibility of the locations of electrodes 102,104, 202, 204 while inserting probes 100, 200 into the bone under surgical imaging, such as fluoroscopy.

As shown in FIGS. 3 and 4, the system also includes a grounding pad 900 which includes the return electrode 902. Grounding pad 900 is connected to radiofrequency generator 1000 via cable 904. Grounding pad 900 may be placed outside the patient and the return electrode 902 may serve as a return electrode for any of the other electrodes 102, 104, 202, 204. Grounding pad 902 may further include a temperature sensor 906.

FIG. 3 shows the ten different configurations that may be utilized for the transmission of radiofrequency energy between the five electrodes 102, 104, 202, 204, 902. As can be seen from the pattern created by the possible radiofrequency energy paths RF, this system provides a surgeon with the ability to ablate any irregularly-shaped tumor without having to relocate probes 100, 200. This provides surgeons with a significant time saving advantage over prior systems, wherein the surgeon had to relocate the tips of the probes to direct the heat to different desired locations.

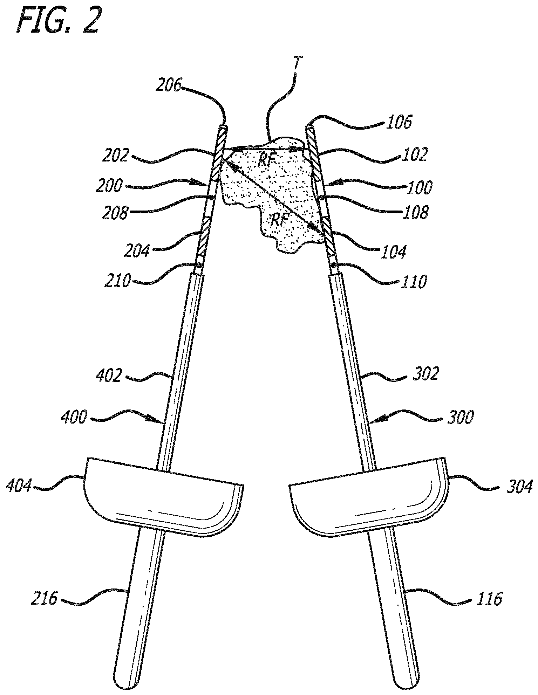

For example, as shown in FIG. 2, an irregularly-shaped tumor T located between probes 100, 200 may be fully ablated without moving probes 100, 200 by first transmitting radiofrequency energy between electrode 102 and electrode 202, followed by transmitting radiofrequency energy between electrode 104 and electrode 202.

The processor controlling the radiofrequency generator may be preprogramed to automatically adjust the amount and duration of radiofrequency energy applied between any pair of electrodes 102, 104, 202, 204, 902 based on temperature data received by the processor from the temperature sensors 106, 108, 110, 206, 208, 210, 906. The surgeon may interact with the processor through a touchscreen 1002. Touchscreen 1002 may also be connected to an imaging device, such as a fluoroscope, to display the target area on touchscreen 1002. The processor may also be preprogrammed to recognize the locations of probes 100, 200 on the screen. This may be accomplished through image recognition technology, aided by the presence of the radiopaque markers adjacent electrodes 102,104, 202, 204. After inserting probes 100, 200 relative to the tumor, the surgeon may outline the tissue to be ablated on the touchscreen and the processor will automatically determine which pairs of electrodes 102, 104, 202, 204, 902 to utilize, the amount of radiofrequency energy to apply to each pair, and the duration of radiofrequency energy application based on the orientation of probes 100, 200, the distance between electrodes 102, 104, 202, 204, 902, the shape and orientation of the tissue to be ablated relative to electrodes 102, 104, 202, 204, 902, and temperature data received from temperature sensors 106, 108, 110, 206, 208, 210, 906.

As shown in FIGS. 1 and 2, the system includes a pair of cannulas 300, 400. Cannulas 300, 400 have an internal diameter slightly larger than an outer diameter of probes 100, 200 to eliminate any lateral movement of probes 100, 200 relative to cannulas 300, 400 when inserted therethrough. Each cannula 300, 400 includes a shaft 302, 402 having a length sufficient to contact the target bone and extend beyond the patient's skin. It is contemplated that different lengths and diameters of probes 100, 200 and cannulas 300, 400 would be utilized for different parts of the body or different- sized patients. Each cannula 300, 400 further includes a handle 304, 404 disposed on the proximal end to aid in the manipulation thereof.

As shown in FIG. 4, the system may include a peristaltic pump 1100 and reservoirs 1102, 1104 attached to probes 100, 200 via tubes 700, 800 for pumping the cooling fluid through probes 100, 200. Each tube 700, 800 includes a first channel leaving the reservoir 1102, 1104 running through peristaltic pump 1100, and coupled to an entrance to the cooling channel on the proximal end of handle 116, 216; and a second channel coupled to an exit of the cooling channel on the proximate end of handle 116, 118 and returning to reservoir 1102, 1104. The system may also include additional standalone temperature sensors 1200 that may be inserted through separate incisions to any desired locations, for example, between the area being treated and a sensitive organ or nerve to prevent damage thereto.

In a preferred embodiment of the present invention, the system is utilized in the following manner. The preferred method includes placing the patient in the prone position and making a pair of contralateral incisions in the skin over the target vertebral body V. The surgeon then inserts cannulas 300, 400 through the incisions into contact with the outer surface of vertebral body V. The surgeon may manipulate the positioning of cannulas 300, 400 by grasping handles 304, 404, preferably under fluoroscopy to verify correct placement of the cannulas.

The surgeon then introduces a bone removal tool, such as a drill or reamer, through cannulas 300, 400 to a desired depth. The desired depth of the bone removal tool may be controlled by utilizing a series of depth markers on the proximal end of the bone removal tool. After creating two openings in the bone, the surgeon inserts probe 100 into one of the openings and inserts probe 200 into the other of the openings. The surgeon then verifies correct placement of probes 100, 200 in relation to the tumor via use of an imaging device. The surgeon then connects cables 500, 600 and tubes 700, 800 to the proximal ends of handles 116, 216 of probes 100, 200. The opposite ends of tubes 700, 800 are attached to reservoirs 1102, 1104 and the opposite ends of cables 500, 600 are attached to radiofrequency generator 1000. The surgeon may also place grounding pad 900 on the outside of the patient's skin.

In a preferred embodiment, the surgeon views the fluoroscope image on touchscreen 1002, showing vertebral body V with probes 100, 200 inserted on opposite sides of the tumor. And the surgeon may then trace the exterior boundary of the tumor on touchscreen 1002. After the surgeon inputs the tumor boundary, the processor runs an algorithm to determine which pair(s) of electrodes 102, 104, 202, 204, 902 should be used, the amount of radiofrequency energy to be applied, and the duration of the application. The processor continually monitors the temperature data received from temperature sensors 106, 108, 110, 206, 208, 210, 906, 1200 and automatically adjusts which electrodes 102, 104, 202, 204, 902 are utilized, the amount of radiofrequency energy applied, and the duration. When the ablation is complete, the radiofrequency generator produces an audible and visual signal to signify completion.

Alternatively, the surgeon can manually select the preferred electrodes 102, 104, 202, 204, 902 to be utilized, the amount of radiofrequency energy applied, and the duration. The surgeon may watch real-time temperature outputs from temperature sensors 106, 108, 110, 206, 208, 210, 906, 1200 and manually make the necessary adjustments.

After completion of the desired ablation, the surgeon removes probes 100, 200. At this point, if indicated, the surgeon may perform vertebroplasty or Kyphoplasty through the already placed cannulas 300, 400. If not indicated, or after performing the vertebroplasty or Kyphoplasty, the surgeon removes cannulas 300, 400 and closes the incisions using an appropriate closure technique.

Other embodiments of the invention will be apparent to those skilled in the art from consideration of the specification and practice of the invention disclosed herein. It is intended that the embodiments described below be considered as exemplary only, with a true scope of the invention being indicated by the appended claims.

According to a first further embodiment, a method of tumor ablation is provided, the method comprising: making a first incision and a second incision in skin of a patient; creating a first opening and a second opening in a bone; inserting a first probe including a first electrode and a second electrode through the first incision and into the first opening; inserting a second probe including a third electrode and a fourth electrode through the second incision and into the second opening; attaching the first and second probes to a radiofrequency generator via a pair of cables; applying radiofrequency energy between a first pair of electrodes, the first pair of electrodes consisting of one of the first and second electrodes and one of the third and fourth electrodes; applying radiofrequency energy between a second pair of electrodes, the second pair of electrodes consisting of any two of the first, second, third, and fourth electrodes, other than the two electrodes included in the first pair; and removing the first and second probes.

According to a second further embodiment, the method of the first further embodiment is provided, further comprising monitoring the temperature in the bone via six temperature sensors disposed on the f probes.

According to a third further embodiment, the method of the second further embodiment is provided, further comprising modifying the amount of radiofrequency energy applied based on the monitored temperatures.

According to a fourth further embodiment, the method of the second or third further embodiment is provided, further comprising modifying which electrodes are applying radiofrequency energy based on the monitored temperatures.

According to a fifth further embodiment, the method of one of the first to fourth further embodiments is provided, further comprising attaching the first probe to a first reservoir via a first tube and attaching the second probe to a second reservoir via a second tube.

According to a sixth further embodiment, the method of the fifth further embodiment is provided, further comprising using a first peristaltic pump to pump fluid from the first reservoir through the first probe, and using a second peristaltic pump to pump fluid from the second reservoir through the second probe.

According to a seventh further embodiment, the method of one of the first to sixth further embodiments, further comprising placing a grounding pad including a return electrode on the outside of the patient.

According to an eighth further embodiment, the method of one of the first to seventh further embodiments is provided, wherein the bone is a vertebral body.

According to a ninth further embodiment, the method of one of the first to eighth further embodiments is provided, further comprising: inserting a first cannula through the first incision in the skin, wherein the creating the first opening in the bone and inserting the first probe are performed through the first cannula; and inserting a second cannula through the second incision in the skin, wherein the creating the second opening in the bone and inserting the second probe are performed through the second cannula.

According to a tenth further embodiment, the method of one of the first to ninth further embodiments is provided, further comprising inserting bone cement into the first and second openings in the bone.

## Claims

1. A system for use in ablation, the system comprising:
a pair of probes (100, 200), each probe (100, 200) having a distal end, a proximal end, and a length between the distal and proximal ends, each probe (100, 200) including three sensors (106, 206, 108, 208, 110, 210), one of which being a distal temperature sensor (106, 206), the other one of which being a midpoint temperature sensor (108, 208) and the remaining one of which being a proximal temperature sensor (110, 210), two electrodes (102, 202, 104, 204), one of which being a distal electrode (102, 202) and the other one of which being a proximal electrode (104, 204), a first insulated section (112, 212), and a second insulated section (114, 214),
wherein, when seen in a length direction of the respective probe (100, 200), the distal temperature sensor (106, 206) is proximate the distal end of the probe (100, 200), the distal electrode (102, 202) is adjacent and proximal of the distal temperature sensor (106, 206), the first insulated section (112, 212) is proximal and adjacent of the distal electrode (102, 202), the proximal electrode (104, 204) is proximal and adjacent of the first insulated section (112, 212), the midpoint temperature sensor (108, 208) is located on the first insulated section (112, 212) between the distal electrode (102, 202) and the proximal electrode (104, 204), and the second insulated section (114, 214) is proximal and adjacent of the proximal electrode (104, 204), wherein the proximal temperature sensor (110, 210) is disposed on the second insulated section (114, 214),
wherein the system further comprises a radiofrequency generator (1000) and a processor, wherein the pair of probes (100, 200) are connected to the same radiofrequency generator (1000), the radiofrequency generator (1000) being configured to transmit radiofrequency energy between any pair of the four electrodes (102, 202, 104, 204), and wherein the six temperature sensors (106, 206, 108, 208, 110, 210) are configured to transmit temperature data to the processor, the processor being configured to control which pair of electrodes (102, 202, 104, 204) is utilized by the radiofrequency generator (1000) and the amount of radiofrequency energy delivered.

2. The system of claim 1, further comprising a grounding pad (900) including a return electrode (902).

3. The system of claim 1 or 2, further comprising a first access cannula (300) configured to pass a first probe (100) of the pair of probes (100, 200) therethrough, and a second access cannula (400) configured to pass a second probe (200) of the pair of probes (100, 200) therethrough.

4. The system of one of claims 1-3, wherein each of the pair of probes (100, 200) further includes an enclosed passage with an entrance and an exit located near the proximal end of the probe (100, 200), the passage traversing a majority of the length of the probe (100, 200).

5. The system of claim 4, further comprising a first tube (700) for connecting one of the enclosed passageways to a reservoir (1102), and a second tube (800) for connecting the other of the enclosed passageways to a second reservoir (1104).

6. The system of one of claims 1-5, further comprising a pair of cables (500, 600) for connecting the electrodes (102, 202, 104, 204) to the radiofrequency generator (1000).

7. The system of one of claims 1-6, wherein each probe (100, 200) further includes radiopaque markers identifying the locations of the electrodes (102, 202, 104, 204).

8. The system of one of claims 1-7, wherein each probe (100, 200) further includes radiopaque markers identifying the locations of the temperature sensors (106, 206, 108, 208, 110, 210).

9. The system of one of claims 1-8, further including a third probe including a seventh temperature sensor (1200).

10. The system of one of claims 1-9, further including a peristaltic pump (1100) to circulate cooling fluid through the pair of probes (100, 200).

## Patentansprüche

1. System zur Verwendung bei einer Ablation, wobei das System umfasst:
ein Sondenpaar (100, 200), wobei jede Sonde (100, 200) ein distales Ende, ein proximales Ende und eine Länge zwischen dem distalen und dem proximalen Ende aufweist, wobei jede Sonde (100, 200) drei Sensoren (106, 206, 108, 208, 110, 210), von denen einer ein distaler Temperatursensor (106, 206) ist, der andere ein in der Mitte befindlicher Temperatursensor (108, 208) ist und der verbleibende ein proximaler Temperatursensor (110, 210) ist, zwei Elektroden (102, 202, 104, 204), von denen eine eine distale Elektrode (102, 202) und die andere eine proximale Elektrode (104, 204) ist, einen ersten isolierten Abschnitt (112, 212) und einen zweiten isolierten Abschnitt (114, 214) einschließt,
wobei der distale Temperatursensor (106, 206), in Längsrichtung der jeweiligen Sonde (100, 200) gesehen, in der Nähe des distalen Endes der Sonde (100, 200) liegt, die distale Elektrode (102, 202) benachbart und proximal zum distalen Temperatursensor (106, 206) liegt, der erste isolierte Abschnitt (112, 212) proximal und benachbart zur distalen Elektrode (102, 202) liegt, die proximale Elektrode (104, 204) proximal und benachbart zum ersten isolierten Abschnitt (112, 212) liegt, der in der Mitte befindliche Temperatursensor (108, 208) sich auf dem ersten isolierten Abschnitt (112, 212) zwischen der distalen Elektrode (102, 202) und der proximalen Elektrode (104, 204) befindet und der zweite isolierte Abschnitt (114, 214) proximal und benachbart zur proximalen Elektrode (104, 204) liegt,
wobei der proximale Temperatursensor (110, 210) auf dem zweiten isolierten Abschnitt (114, 214) angeordnet ist, wobei das System ferner einen Hochfrequenzgenerator (1000) und einen Prozessor umfasst, wobei das Sondenpaar (100, 200) mit demselben Hochfrequenzgenerator (1000) verbunden ist, wobei der Hochfrequenzgenerator (1000) konfiguriert ist, um Hochfrequenzenergie zwischen einem beliebigen Paar der vier Elektroden (102, 202, 104, 204) zu übertragen, und wobei die sechs Temperatursensoren (106, 206, 108, 208, 110, 210) konfiguriert sind, um Temperaturdaten an den Prozessor zu übertragen, wobei der Prozessor konfiguriert ist, um zu steuern, welches Elektrodenpaar (102, 202, 104, 204) vom Hochfrequenzgenerator (1000) verwendet wird und wie viel Hochfrequenzenergie abgegeben wird.

2. System nach Anspruch 1, ferner umfassend eine Neutralelektrode (900) mit einer Gegenelektrode (902).

3. System nach Anspruch 1 oder 2, ferner umfassend eine erste Zugangskanüle (300), die konfiguriert ist, um eine erste Sonde (100) des Sondenpaars (100, 200) dorthindurch zu führen, und eine zweite Zugangskanüle (400), die konfiguriert ist, um eine zweite Sonde (200) des Sondenpaars (100, 200) dorthindurch zu führen.

4. System nach einem der Ansprüche 1-3, wobei jedes der Sondenpaare (100, 200) ferner einen umschlossenen Durchgang mit einem Eingang und einem Ausgang enthält, der sich nahe dem proximalen Ende der Sonde (100, 200) befindet, wobei der Durchgang den Großteil der Länge der Sonde (100, 200) durchquert.

5. System nach Anspruch 4, ferner umfassend ein erstes Rohr (700) zum Verbinden von einem der umschlossenen Durchgänge mit einem Vorratsbehälter (1102) und ein zweites Rohr (800) zum Verbinden des anderen der umschlossenen Durchgänge mit einem zweiten Vorratsbehälter (1104).

6. System nach einem der Ansprüche 1-5, ferner umfassend ein Kabelpaar (500, 600) zum Verbinden der Elektroden (102, 202, 104, 204) mit dem Hochfrequenzgenerator (1000).

7. System nach einem der Ansprüche 1-6, wobei jede Sonde (100, 200) ferner röntgendichte Marker enthält, die die Positionen der Elektroden (102, 202, 104, 204) identifizieren.

8. System nach einem der Ansprüche 1-7, wobei jede Sonde (100, 200) ferner röntgendichte Marker enthält, die die Positionen der Temperatursensoren (106, 206, 108, 208, 110, 210) identifizieren.

9. System nach einem der Ansprüche 1-8, ferner einschließend eine dritte Sonde mit einem siebten Temperatursensor (1200).

10. System nach einem der Ansprüche 1-9, ferner einschließend eine Peristaltikpumpe (1100), um Kühlflüssigkeit durch das Sondenpaar (100, 200) zu zirkulieren.

## Revendications

1. Système destiné à être utilisé dans l'ablation, le système comprenant :
une paire de sondes (100, 200), chaque sonde (100, 200) ayant une extrémité distale, une extrémité proximale et une longueur entre les extrémités distale et proximale, chaque sonde (100, 200) comportant trois capteurs (106, 206, 108, 208, 110, 210), l'un étant un capteur de température distal (106, 206), l'autre étant un capteur de température central (108, 208) et le dernier étant un capteur de température proximal (110, 210), deux électrodes (102, 202, 104, 204), l'une étant une électrode distale (102, 202) et l'autre étant une électrode proximale (104, 204), une première section isolée (112, 212), et une seconde section isolée (114, 214),
dans lequel, lorsqu'il est vu dans la direction de la longueur de la sonde respective (100, 200), le capteur de température distal (106, 206) est proche de l'extrémité distale de la sonde (100, 200), l'électrode distale (102, 202) est adjacente et proximale au capteur de température distal (106, 206), la première section isolée (112, 212) est proximale et adjacente à l'électrode distale (102, 202), l'électrode proximale (104, 204) est proximale et adjacente à la première section isolée (112, 212), le capteur de température central (108, 208) est situé sur la première section isolée (112, 212) entre l'électrode distale (102, 202) et l'électrode proximale (104, 204), et la seconde section isolée (114, 214) est proximale et adjacente à l'électrode proximale (104, 204), dans lequel le capteur de température proximal (110, 210) est disposé sur la seconde section isolée (114, 214),
le système comprenant en outre un générateur de radiofréquence (1000) et un processeur, dans lequel la paire de sondes (100, 200) sont connectées au même générateur de radiofréquence (1000), le générateur de radiofréquence (1000) étant configuré pour transmettre de l'énergie radiofréquence entre n'importe quelle paire des quatre électrodes (102, 202, 104, 204), et dans lequel les six capteurs de température (106, 206, 108, 208, 110, 210) sont configurés pour transmettre des données de température au processeur, le processeur étant configuré pour contrôler quelle paire d'électrodes (102, 202, 104, 204) est utilisée par le générateur de radiofréquence (1000) et la quantité d'énergie radiofréquence délivrée.

2. Système selon la revendication 1, comprenant en outre une borne de masse (900) comportant une électrode de retour (902).

3. Système selon la revendication 1 ou 2, comprenant en outre une première canule d'accès (300) conçue pour faire passer une première sonde (100) de la paire de sondes (100, 200) à travers celle-ci, et une seconde canule d'accès (400) conçue pour faire passer une deuxième sonde (200) de la paire de sondes (100, 200) à travers celle-ci.

4. Système selon l'une des revendications 1 à 3, dans lequel chacune de la paire de sondes (100, 200) comporte en outre un passage fermé doté d'une entrée et une sortie situées près de l'extrémité proximale de la sonde (100, 200), le passage traversant une majorité de la longueur de la sonde (100, 200).

5. Système selon la revendication 4, comprenant en outre un premier tube (700) pour relier l'un des passages fermés à un réservoir (1102), et un second tube (800) pour relier l'autre des passages fermés à un second réservoir (1104).

6. Système selon l'une des revendications 1 à 5, comprenant en outre une paire de câbles (500, 600) pour relier les électrodes (102, 202, 104, 204) au générateur de radiofréquence (1000).

7. Système selon l'une des revendications 1 à 6, dans lequel chaque sonde (100, 200) comporte en outre des marqueurs radio-opaques identifiant les emplacements des électrodes (102, 202, 104, 204).

8. Système selon l'une des revendications 1 à 7, dans lequel chaque sonde (100, 200) comporte en outre des marqueurs radio-opaques identifiant les emplacements des capteurs de température (106, 206, 108, 208, 110, 210).

9. Système selon l'une des revendications 1 à 8, comportant en outre une troisième sonde comportant un septième capteur de température (1200).

10. Système selon l'une des revendications 1 à 9, comportant en outre une pompe péristaltique (1100) destinée à faire circuler le fluide de refroidissement à travers la paire de sondes (100, 200).
